# FASCICULE DE BREVET EUROPEEN

(11) **EP 3 402 397 B1**
(45) Date de publication et mention de la délivrance du brevet: **26.01.2022**
(21) Numéro de dépôt: 17703031.9
(22) Date de dépôt: 09.01.2017
(51) Int. Cl.: A61B 5/00, A61N 1/37, A61B 5/24, A61B 5/346

(54) **DISPOSITIF MEDICAL IMPLANTABLE ACTIF DE DEFIBRILLATION, COMPRENANT DES MOYENS DE DISCRIMINATION ENTRE BRUIT EXTERNE ET RUPTURE DE SONDE AINSI QUE DES MOYENS DE CARACTERISATION DES TACHYARYTHMIES**
AKTIVE IMPLANTIERBARE MEDIZINISCHE DEFIBRILLATIONSVORRICHTUNG MIT VERBESSERTEN MITTELN ZUR UNTERSCHEIDUNG ZWISCHEN EXTERNEM SCHALL UND SONDENBRUCH UND ZUR CHARAKTERISIERUNG VON TACHYARRHYTHMIEN
ACTIVE IMPLANTABLE MEDICAL DEFIBRILLATION DEVICE, INCLUDING IMPROVED MEANS FOR DISCRIMINATING BETWEEN EXTERNAL NOISE AND PROBE BREAKAGE AND FOR CHARACTERISING TACHYARRHYTHMIAS

(30) Priorité: 11.01.2016 FR 1650194
(43) Date de publication de la demande: 21.11.2018
(73) Titulaire: Sorin CRM SAS, 92140 Clamart (FR)
(72) Inventeur: EUZEN, Marie-Anne, 91570 Bièvres (FR); FEUERSTEIN, Delphine, 92130 Issy les Moulineaux (FR)
(74) Mandataire: Grünecker Patent- und Rechtsanwälte PartG mbB
(86) Numéro de dépôt international: PCT/EP2017/050345
(87) Numéro de publication internationale: WO 2017/121702

(56) Documents cités:
- EP-A1- 2 368 493
- US-A- 5 404 880
- US-A1- 2010 280 567
- US-A1- 2013 030 481
- US-A1- 2015 224 321
- US-A1- 2015 251 012

## Description

L'invention concerne les "dispositifs médicaux implantables actifs" tels que définis par la directive 90/385/CEE du 20 juin 1990 du Conseil des communautés européennes, plus précisément les implants permettant de surveiller en continu le rythme cardiaque et délivrer si nécessaire au cœur des impulsions électriques de stimulation, de resynchronisation et/ou de défibrillation en cas de trouble du rythme détecte par le dispositif. L'invention est plus particulièrement destinée aux appareils permettant de délivrer au cœur des thérapies antitachycardiques impliquant l'application contrôlée de chocs de défibrillation (impulsions électriques de haute énergie destinées à mettre fin à une tachyarythmie) et/ou de thérapies par stimulation à haute fréquence de type dit ATP (*AntiTachycardia Pacing*)*.* Une difficulté tient au fait que ces dispositifs sont sensibles à la détection de signaux externes d'origine non cardiaque, dus à des problèmes liés à la sonde, à des interférences électromagnétiques, à la détection de myo-potentiels, etc., phénomènes ci-après désignés sous le terme générique de "bruit".

Ces phénomènes sont susceptibles de leurrer le dispositif, pouvant en-trainer des conséquences très graves, par exemple en inhibant à tort des stimulations antibradycardiques ou bien, inversement, en commandant la délivrance de chocs de défibrillation inappropriés, du fait de la "surdétection" de signaux extracardiaques sans aucune relation avec des tachyarythmies ventriculaires.

Or, l'application d'un choc de défibrillation chez un patient conscient est extrêmement douloureuse et angoissante, les énergies appliquées étant en effet très au-delà du seuil de la douleur. En outre, l'application d'un choc de défibrillation n'est pas dénuée d'effets secondaires sur le rythme cardiaque (risque d'apparition de troubles secondaires), sur l'intégrité fonctionnelle du myocarde, et de façon générale sur l'équilibre physiologique du patient. Il est donc important de ne délivrer de tels chocs que de façon appropriée.

Diverses techniques ont été proposées pour filtrer ou discriminer *a priori* les phénomènes de bruit extracardiaque, avant la détection ventriculaire, ou pour analyser a *posteriori* le signal recueilli afin de déterminer si celuici est ou non potentiellement parasité par du bruit, et modifier si besoin le fonctionnement du dispositif en cas de suspicion de bruit.

Ainsi, le EP 2 368 493 A1 (Sorin CRM) propose une méthode de détection et d'élimination du bruit ventriculaire basée sur l'analyse bidimensionnelle de deux signaux d'électrogramme endocavitaire (EGM) recueillis concurremment sur deux voies distinctes. Dans ce document, les deux voies distinctes sont des voies provenant de la même cavité, en l'espèce le ventricule droit, mais de façon générale la technique peut aussi être mise en œuvre à partir d'EGMs provenant de deux cavités différentes.

Les deux voies EGM différentes peuvent être en particulier celles d'un signal unipolaire (signal lointain recueilli entre le boitier et une électrode distale ou bien proximale) et celle d'un signal bipolaire (signal proche recueilli entre une électrode distale et une électrode proximale), respectivement. L'analyse bidimensionnelle est effectuée sur une "boucle cardiaque" ou "vectogramme" (VGM), qui est la représentation dans un espace à deux dimensions de l'un des deux signaux par rapport à l'autre, chaque battement cardiaque ou fraction significative de battement étant alors représenté par son vectogramme dans le plan ainsi défini.

Plus précisément, le procédé d'analyse consiste à décrire le VGM par un paramètre descripteur de la morphologie intrinsèque de celui-ci, typiquement l'angle moyen que forment les vecteurs vitesse consécutifs le long du parcours de la boucle du VGM. L'analyse de ce descripteur morphologique permet de déterminer ensuite si le battement courant est d'origine cardiaque, ou s'il s'agit de bruit. Le choix de ce descripteur particulier est basé sur l'hypothèse que, pour un véritable complexe cardiaque, deux vecteurs consécutifs ne doivent pas trop changer de direction tandis que, dans le cas d'un bruit, deux vecteurs vitesse consécutifs peuvent aller dans des directions très différentes, voire opposées.

Diverses actions peuvent être entreprises selon que l'on est en présence de cycles valides ou invalides, notamment:
- effacement des marqueurs correspondent aux cycles invalides bruités ;
- modification éventuelle de la sensibilité des circuits de détection ou autre paramètre jouant sur la détection ;
- inhibition ou temporisation d'une thérapie ;
- modification du marquage d'identification des épisodes de tachyarythmie ;
- allongement des périodes réfractaires ; et/ou
- effacement des épisodes invalides stockés dans la mémoire.

Les essais cliniques montrent que la mise en œuvre de la méthode décrite par le EP 2 368 493 A1, avec un descripteur correspondant à l'angle moyen entre deux vecteurs vitesse consécutifs, procure une spécificité de détection proche de 100 %.

En revanche, la sensibilité de détection n'est pas optimale, et dépend de l'origine du bruit: ainsi, dans le cas d'une rupture de sonde, comme le bruit est présent dans les deux signaux utilisés pour la construction du VGM, le vectogramme progresse de façon à peu près continue et la variation de l'angle du vecteur vitesse peut ne pas être suffisante pour conclure à du bruit, et/ou ne présenter une variation importante que sur un faible nombre de cycles, ne permettant pas de tirer une conclusion et donc ne permettant pas d'inhiber une thérapie et/ou d'alerter le médecin.

Le but de la présente invention est de remédier à cette difficulté, en proposant un perfectionnement à la méthode ci-dessus, divulguée par le EP 2 368 493 A1 précité, qui permette d'augmenter en toute sécurité la sensibilité de détection du bruit tout en évitant les risques de surdétection, notamment du fait d'une rupture de sonde, afin de pouvoir prendre en toute sécurité et sans délai les actions qui s'imposent en fonction de l'origine du bruit.

Subsidiairement, comme on le verra, un autre but de l'invention est, en l'absence de bruit détecte, c'est-à-dire dans le cas d'un cycle valide, de permettre une analyse de l'épisode de tachyarythmie distinguant entre tachyarythmie monomorphe et tachyarythmie polymorphe.

Plus précisément, l'invention propose un dispositif selon la revendication indépendante 1.

Selon diverses caractéristiques subsidiaires avantageuses :
- les classes correspondent à des intervalles d'amplitude contigus et égaux;
- le critère prédéterminé est la présence de deux classes non vides séparées par un intervalle prédéterminé de classes vides consécutives, et l'indicateur est un indicateur de suspicion d'artefact par bruit d'origine extraventriculaire, l'intervalle prédéterminé pouvant être notamment un intervalle correspondent à un écart entre classes non vides d'au moins 10 mV;
- le critère prédéterminé est la présence d'au moins une classe non vide correspondent à un intervalle d'amplitude supérieur à une première valeur limite donnée, et l'indicateur est un indicateur de suspicion d'artefact per rupture de sonde, ladite première valeur limite pouvant être notamment d'au moins 20 mV ;
- le critère prédéterminé est le fait que, en l'absence de détection d'artefact par les moyens de détection d'artefacts d'origine extracardiaque, toutes les classes non vides sont des classes correspondant à des intervalles d'amplitude inférieure à une deuxième valeur limite donnée, inférieure à ladite première valeur limite, et l'indicateur est un indicateur de tachyarythmie monomorphe, ladite deuxième valeur limite pouvant être notamment d'au moins 2 mV ;
- le critère prédéterminé est le fait que, en l'absence de détection d'artefact par les moyens de détection d'artefacts d'origine extracardiaque, toutes les classes non vides sont des classes correspondant à des intervalles d'amplitude inférieure à une troisième valeur limite donnée, supérieure à ladite deuxième valeur limite et inférieure à ladite première valeur limite, et l'indicateur est un indicateur de tachyarythmie polymorphe, ladite troisième valeur limite pouvant être notamment d'au moins 5 mV.

On va maintenant décrire un exemple de mise en œuvre de la présente invention, en référence aux dessins annexés où les mêmes références désignent d'une figure à l'autre des éléments identiques ou fonctionnellement semblables.

La Figure 1 est une vue générale montrant un dispositif de stimulation/ défibrillation avec son générateur et une sonde implantée dans le cœur droit.
La Figure 2 est un exemple de signaux EGM obtenus sur des voies respectivement ventriculaire bipolaire et ventriculaire unipolaire de l'une des sondes de la Figure 1.
La Figure 3 illustre la manière de combiner entre eux les signaux bipolaire et unipolaire recueillis dans une même cavité ventriculaire pour construire une caractéristique bidimensionnelle de type vectogramme, indépendante du temps.
La Figure 4 est un exemple de vectogramme échantillonné obtenu pour un cycle cardiaque échantillonné à 128 Hz, avec représentation des vecteurs vitesse en divers points successifs.
La Figure 5 illustre schématiquement les différentes étapes de mise en œuvre de la méthode de détection et de gestion du bruit selon l'invention.
Les Figures 6a et 6b illustrent des histogrammes obtenus par mise en œuvre de la méthode de l'invention, dans différentes situations où un bruit externe est avéré.
Les Figures 7a et 7b illustrent des histogrammes obtenus par mise en œuvre de la méthode de l'invention, dans différentes situations de tachyarythmie où aucun bruit externe n'est détecté.
La Figure 8 illustre schématiquement les différentes étapes d'une analyse des profils d'arythmie à partir des histogrammes tels que ceux illustrés Figure 5 et Figure 6.
La Figure 9 illustre schématiquement les différentes étapes d'une analyse conduisant à conclure à une rupture de sonde.

Nous allons maintenant décrire un exemple de réalisation du dispositif de l'invention.

En ce qui concerne ses aspects logiciels, l'invention peut être mise en œuvre par une programmation appropriée du logiciel de commande d'un dispositif connu comprenant des moyens d'acquisition d'un signal fourni par des sondes endocavitaires et/ou un ou plusieurs capteurs implantés, et des moyens d'application d'une thérapie antitachycardique (choc de défibrillation et/ou stimulation ATP).

L'invention peut notamment être appliquée aux dispositifs implantables tels que ceux de la famille *Paradym* ou *Platinum* produits et commercialisés par Sorin CRM, Clamart, France.

Il s'agit de dispositifs à microprocesseur programmable comportant des circuits pour recevoir, mettre en forme et traiter des signaux électriques recueillis par des électrodes implantées, et délivrer des impulsions de stimulation et de défibrillation à ces électrodes. Il est possible d'y transmettre par télémétrie des logiciels qui seront conservés en mémoire et exécutés pour mettre en œuvre les fonctions de l'invention qui seront décrites ci-dessous. L'adaptation de ces appareils à la mise en œuvre des fonctions de l'invention est à la portée de l'homme du métier, et elle ne sera pas décrite en détail.

Le procédé de l'invention est mis en œuvre par des moyens principalement logiciels, au moyen d'algorithmes appropriés exécutés par un micro-contrôleur ou un processeur numérique de signal. Pour la clarté de l'exposé, les divers traitements appliqués seront décomposés et schématisés par un certain nombre de blocs fonctionnels distincts présentés sous forme de circuits interconnectés, mais cette représentation n'a toutefois qu'un caractère illustratif, ces circuits comprenant des éléments communs et correspondent en pratique à une pluralité de fonctions globalement exécutées par un même logiciel.

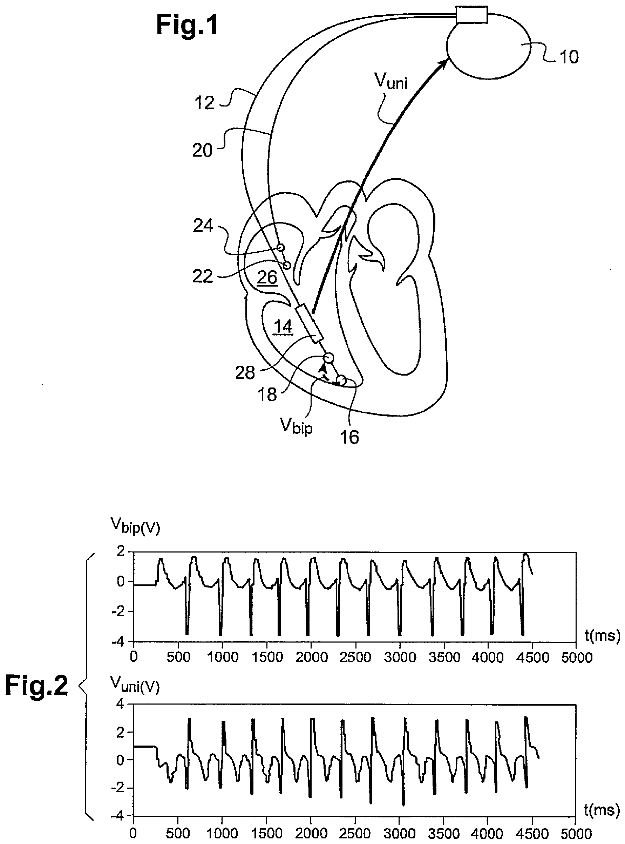

La Figure 1 illustre une configuration typique de stimulation "double-chambre" dans laquelle un générateur d'impulsions 10 est associé à une première sonde 12 implantée dans le ventricule droit 14. La tête de cette sonde comporte deux électrodes, à savoir une électrode distale *(tip)* 16 et une électrode proximale *(ring)* 18. Une éventuelle deuxième sonde 20 est pourvue d'électrodes auriculaires de détection distale 22 et proximale 24 situées au niveau de l'oreillette droite 26 pour la détection des signaux dans cette cavité et l'application éventuelle d'une stimulation auriculaire.

La sonde ventriculaire droite 12 est également pourvue d'un bobinage *(coil)* ventriculaire 28 formant électrode de défibrillation et permettant aussi de recueillir un signal endocavitaire (ce bobinage pouvant alors être utilisé à la place de l'électrode proximale *ring* 18).

Si le dispositif assure en outre des fonctions de resynchronisation ventriculaire (stimulation CRT, *Cardiac Resynchronization Therapy),* il est également prévu une sonde de stimulation du ventricule gauche, typiquement une sonde introduite par le réseau veineux coronaire.

En premier lieu, il convient de combiner deux signaux d'électrogramme endocavitaire recueillis en rythme spontané du patient, en particulier des signaux issus de la même cavité ventriculaire, par exemple du ventricule droit.

Les EGMs recueillis à cet effet dans le ventricule droit peuvent comprendre par exemple (voir Figure 1):
- une composante ventriculaire droite *Vbip,* dérivée d'un signal EGM *near-field* bipolaire recueilli entre l'électrode distale 16 et l'électrode proximale 18 de la sonde ventriculaire droite 12, et
- une autre composante ventriculaire droite *Vuni,* dérivée d'un signal EGM *far-field* unipolaire recueilli entre le bobinage de défibrillation 28 de la sonde ventriculaire droite 12 et le boitier métallique du générateur 10.

D'autres configurations peuvent être utilisées, à partir de signaux de type *far-field* (par exemple entre l'une des électrodes 16 ou 18 et le boitier 10) et de type *near-field* (entre deux électrodes de la même sonde ventriculaire).

La Figure 2 illustre un exemple de traces d'EGMs *Vbip* et *Vuni* observés respectivement sur la voie bipolaire ventriculaire et la voie unipolaire ventriculaire de la configuration de la Figure 1.

Ces deux signaux bipolaire et unipolaire sont combinés en une caractéristique unique de type "boucle cardiaque" ou "vectogramme" (VGM), qui est la représentation dans un espace à deux dimensions de l'un des deux signaux EGM (en ordonnée) par rapport à l'autre (en abscisse). Chaque cycle cardiaque est alors représenté par un vectogramme dans le plan *{Vbip, Vuni}* ainsi défini, vectogramme dont la géométrie (forme de la courbe) fait donc abstraction de la dimension temporelle - qui n'intervient que comme un paramètre décrivant la manière dont la courbe est parcourue.

On soulignera que ce "vectogramme" (VGM), qui est obtenu à partir de signaux d'électrogramme (EGM) issu de sondes intracardiaques, ne doit pas être confondu avec le "vectocardiogramme" (VCG) qui est, lui, obtenu à partir de signaux d'électrocardiogramme (EGG) issu d'électrodes externes placées sur le thorax du patient.

On notera également que l'analyse 'bidimensionnelle" ou "en deux dimensions" (2D) évoquée ici ne doit pas être entendue de manière en elle-même limitative. L'invention peut en effet s'appliquer aussi bien à une analyse dans un espace multidimensionnel d'ordre supérieur (3D ou plus), par extrapolation des enseignements de la présente description à une situation où des signaux EGM provenant d'une même cavité sont recueillis simultanément sur trois voies ou plus.

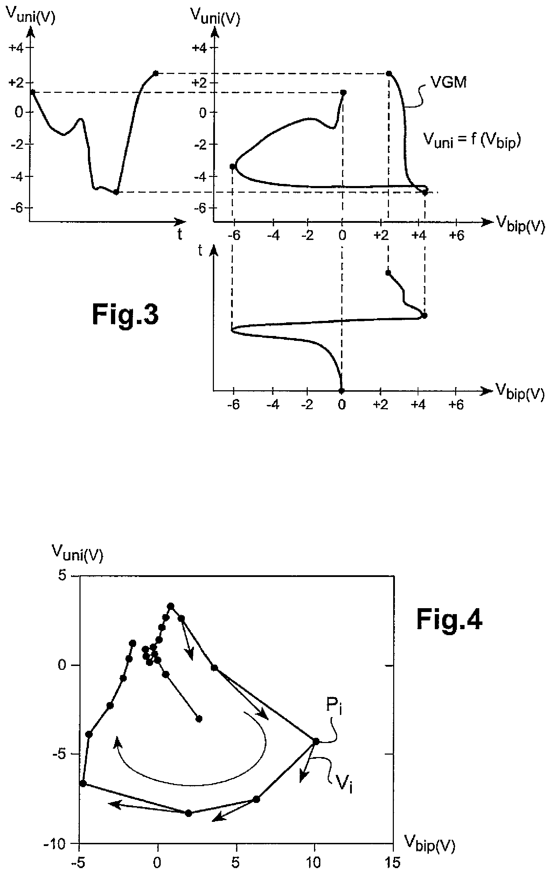

Comme illustré Figure 3, les signaux EGM *Vuni(t)* et *Vbip(t)* recueillis sont échantillonnés, et les échantillons successifs des deux composantes sont mémorisés puis combinés entre eux pour produire une courbe paramétrique (la caractéristique VGM) du type VGM = (*Vbip*(t)*,Vuni*(t)) ou {x = *Vbip*(t), y = *Vuni*(t)}.

En d'autres termes, cette courbe est une courbe paramétrée par le temps, tracée à partir des variations de l'une des composantes temporelles *(Vuni)* en fonction de l'autre *(Vbip).* Elle constitue un vectogramme (VGM) représentatif du cycle cardiaque à analyser, et sera également désignée "caractéristique 2D paramétrique". Elle présente graphiquement la forme d'une boucle, le temps n'apparaissant plus que dans la manière dont la boucle est parcourue sur la durée du cycle.

En pratique, comme illustré Figure 4, l'échantillonnage produit un VGM en forme de polygone ouvert où chaque sommet correspond à un point d'échantillonnage de la mesure du signal *Vuni* et *Vbip* de l'EGM. Dans l'exemple de la Figure 4, l'échantillonnage est opéré à une fréquence de 128 Hz, ce qui donne environ 11 points de mesure pour un intervalle de temps de 80 ms, qui sont autant de valeurs qui peuvent être mises en mémoire pour être analysées.

On a également représenté sur la Figure 4 l'allure du vecteur vitesse V en divers points successifs Pᵢ du VGM pour une fréquence d'échantillonnage de 128 Hz. En un point donné, la vitesse est une donnée vectorielle (la vitesse étant définie par son orientation et sa norme), et le vecteur vitesse peut être calculé en chaque point du VGM à partir d'un filtre discret qui approxime les dérivées premières dV*bip*(t)/dt et dV*uni*(t)/dt qui, pour une caractéristique échantillonnée, peuvent être calculées à partir du point précèdent et du point suivant sur la courbe.

La caractéristique VGM recueillie est mémorisée sous forme d'une série de paramètres descripteurs bases sur les vecteurs vitesse en chaque point de la courbe et comprenant la norme du vecteur vitesse et l'orientation de ce vecteur vitesse, c'est-à-dire l'angle qu'il fait par rapport à l'axe des abscisses du VGM.

On va maintenant exposer, dans ce contexte, les aspects spécifiques de l'invention.

L'idée de base de la présente invention consiste essentiellement, lors d'un épisode de tachyarythmie, à combiner:
- l'analyse du descripteur morphologique du VGM (c'est-à-dire la variation de l'angle moyen entre deux vecteurs vitesse V consécutifs, variation ci-après désignée "ΔV"), enseignée par le EP 2 368 493 A1 précité, avec
- une analyse de la variation de l'amplitude A (variation ci-après désignée "ΔA") de l'un des deux signaux EGM qui est utilisé pour la détection ventriculaire, de préférence de l'EGM bipolaire, le suivi de la variation d'amplitude cycle-à-cycle se faisant par calcul de la différence AA entre l'amplitude "crête-a-crête" A du battement courant et celle du battement précédent.

Pour chaque battement courant, on calcule ainsi une valeur d'angle moyen entre deux vecteurs vitesse consécutifs, descripteur ci-après désigné ΔV.

Ce descripteur ΔV reflète la progression du vectogramme pour un cycle donné, progression qui peut être relativement continue (ΔV est alors inférieur à un seuil donné) pour un complexe cardiaque véritable, ou au contraire beaucoup plus erratique (conduisant à une valeur beaucoup plus élevée du descripteur ΔV).

Dans le dernier cas, une différence d'amplitude ΔA trop importante entre deux cycles consécutifs (par exemple ΔA > 20 mV) sera considérée comme non physiologique, et permettra de conclure à un problème de sonde, conduisant à suspendre au moins provisoirement la délivrance d'une thérapie.

Plus précisément, pour permettre une analyse fine du paramètre ΔA, on mémorise sur un nombre prédéterminé de cycles les valeurs successives de la différence d'amplitude ΔA entre un cycle courant et le cycle précèdent, et les valeurs conservées font ensuite l'objet d'une distribution en une pluralité de classes préalablement définies, chaque classe correspondent à un Intervalle d'amplitude donné.

Ces différentes classes Ci correspondent de préférence à des intervalles d'amplitude ΔA contigus et égaux.

Per exemple, si l'on choisit des classes correspondant à un intervalle de 1 mV, on aura :
- une première classe C1 regroupent les valeurs de ΔA comprises entre 0 et 1 mV,
- une deuxième classe C2 regroupent les valeurs de ΔA comprises entre 1 et 2 mV,
- une troisième classe C3 regroupent les valeurs de ΔA comprises entre 2 et 3 mV,
- et ainsi de suite.

Pour la suite de l'analyse, il sera nécessaire de garder seulement en mémoire la définition des classes Ci et le nombre Ni de cycles cardiaques pour lesquels le valeur AA correspond à cette classe Ci.

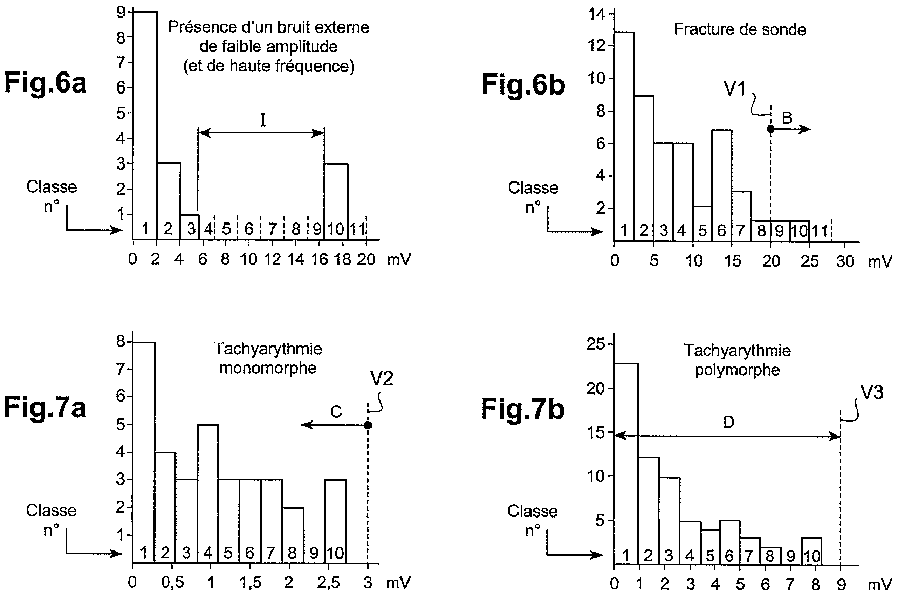

La distribution des valeurs ΔA entre les différentes classes peut être représentée visuellement sous forme d'histogrammes tels que ceux illustrés aux exemples des Figures 6a, 6b, 7a et 7b, qui seront décrites plus en détail per le suite.

La distribution des valeurs de ΔA, et donc le profil de l'histogramme, permet, selon l'invention, de produire des informations supplémentaires :
i) soit sur la présence éventuelle d'un bruit externe ou une situation de rupture de sonde, ou
ii) soit, en l'absence de bruit, sur le nature particulière de la tachyarythmie - monomorphe ou polymorphe.

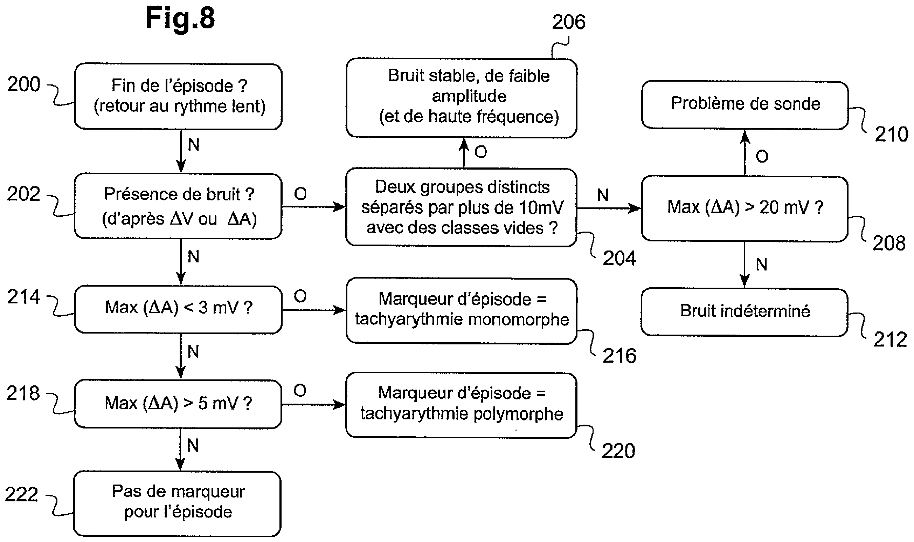

On expliquera plus bas en détail, en référence notamment à l'organigramme de la Figure 8, la manière d'analyser cette distribution des valeurs ΔA pour en dériver ces informations supplémentaires spécifiques.

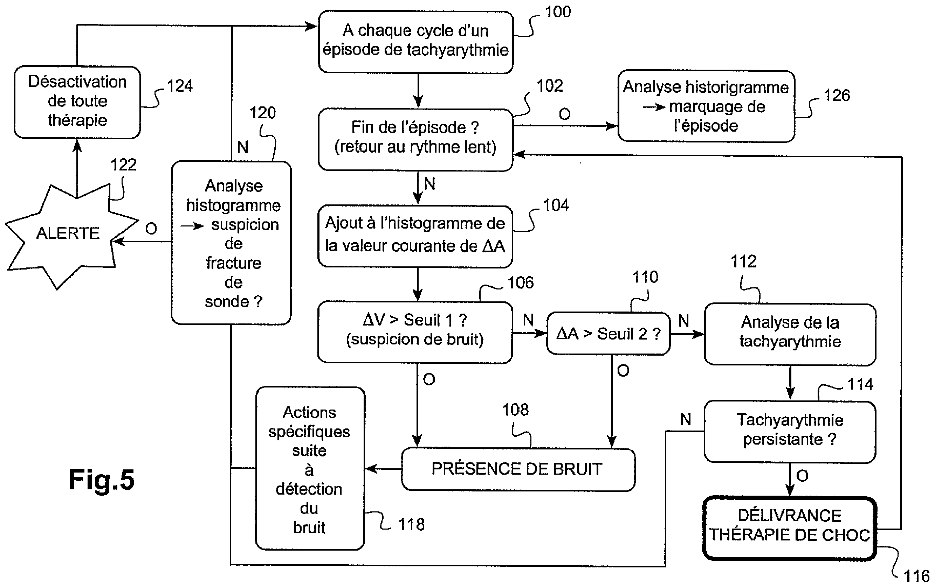

La Figure 5 illustre schématiquement les différentes étapes de mise en œuvre de la méthode selon l'invention.

Les différents tests et actions de cette méthode sont exécutés lorsque le patient est en situation de tachyarythmie, à chaque cycle de cet épisode (bloc 100).

La première étape consiste à détecter un éventuel retour du rythme cardiaque au rythme sinusal lent, qui signifierait la fin de l'épisode de tachyarythmie (bloc 102).

Dans la négative, c'est-à-dire si le patient est toujours en tachyarythmie, la différence d'amplitude ΔA entre le cycle courant et le cycle précèdent est calculée et classifiée, c'est-à-dire que la classe correspondante Ci de l'histogramme est incrémentée d'une unité (bloc 104).

L'étape suivante consiste, de manière en elle-même connue (notamment d'après le EP 2 368 493 A1 précité) à évaluer la variation ΔV de l'angle moyen du vecteur vitesse pour le VGM courant, et à comparer cette variation ΔV à un premier seuil Seuil1 (bloc 106).

Une réponse positive, révélant un parcours relativement erratique du vectogramme pendant le cycle cardiaque, est considérée comme une indication de la présence d'un bruit, et un indicateur spécifique est positionne (bloc 108). Dans le cas contraire, et de façon caractéristique de l'invention, on ne conclut pas à une absence de bruit mais on effectue un second test, basé sur la différence d'amplitude ΔA entre le cycle courant et le cycle précèdent, cette valeur de ΔA étant comparée à un second seuil (seuil 2) (bloc 110):
- si la variation d'amplitude est supérieure au seuil, alors on confirme la présence d'un bruit (bloc 108);
- dans le cas contraire, on considère que l'on est en présence d'un signal cardiaque non bruité, qui peut être analysé par application d'un algorithme usuel de classification des arythmies (bloc 112), par exemple l'algorithme *Parad* des dispositifs conçus par Sorin CRM, décrit dans le EP 0 838 235 A1 (ELA Medical).

Si l'analyse de la tachyarythmie révèle que celle-ci est persistante (bloc 114), alors il convient d'appliquer (éventuellement si d'autres conditions sont réunies après analyse de l'arythmie) une thérapie, typiquement par application d'un choc de défibrillation (bloc 116). Ensuite, le processus retourne au bloc 102 pour vérifier que la thérapie appliquée a bien mis fin à L'épisode de tachyarythmie.

En l'absence de tachyarythmie persistante détectée au bloc 114, on procède alors à une analyse de l'histogramme (bloc 120, décrit ci-après).

S'il y a lieu, un indicateur de présence de bruit a été positionne à l'étape 108, et le dispositif prend alors un certain nombre d'actions spécifiques, propres à la détection d'un bruit. Parmi ces actions, on peut citer les suivantes :
- ajustement de la sensibilité ;
- inhibition de la thérapie ;
- délivrance éventuelle d'une stimulation ventriculaire.

Concernant plus précisément ce dernier point, on remarquera que si le patient est dépendant (bloc auriculoventriculaire) et que le dispositif détecte du bruit, le dispositif ne stimulera pas au bon moment, ce qui produira une pause ventriculaire plus ou moins longue. Or, une stimulation à un instant vulnérable (notamment pendant l'onde T de repolarisation) est plus dangereuse d'une pause ventriculaire ; mais inversement, dans certaines situations, si un rythme sinusal est absent une stimulation sera nécessaire et sans danger.

Pour identifier la présence ou non d'un tel rythme (présence ou non d'un battement cardiaque) à l'aide du signal EGM ventriculaire droit uniquement, il est nécessaire que l'amplitude du bruit soit faible.

De fait, si le cycle a été considéré comme un cycle bruité (présence de bruit signalée au bloc 108):
- si l'amplitude crête-à-crête A du battement courant est inférieure à un seuil donné (par exemple inférieure à 1 mV, ou inférieure à une fraction de l'amplitude moyenne en rythme sinusal), alors l'intervalle d'échappement du dispositif n'est pas réinitialisé, et une stimulation ventriculaire est appliquée à la fin de cet intervalle ;
- en revanche, si cette amplitude A est supérieure au seuil, alors l'intervalle d'échappement est réinitialisé, pour éviter une stimulation à un instant inapproprié qui pourrait être un facteur déclenchant d'une arythmie.

D'autres signaux que l'EGM ventriculaire droit bipolaire peuvent être utilisés, le cas échéant, pour déterminer si une stimulation est nécessaire en cas de bruit détecté. Ainsi :
- pour les patients équipés d'un dispositif resynchroniseur disposant d'une sonde ventriculaire gauche, on peut décider de ne pas réinitialiser l'intervalle d'échappement ventriculaire tant qu'il n'y a pas de dépolarisation du ventricule gauche détectée ;
- pour les patients disposant d'une sonde pourvue d'un capteur d'accélération endocardiaque (EA) implanté, par exemple une sonde pourvue d'un accéléromètre intègre en bout de sonde, l'intervalle d'échappement ventriculaire n'est pas réinitialisé tant que l'on ne détecte pas de composante EA, c'est-à-dire de composante révélant une contraction mécanique du ventricule.

Une fois exécutées les actions spécifiques en présence de bruit (bloc 118) ou, en l'absence de bruit, après confirmation de la persistance de l'épisode de tachyarythmie (bloc 114), de façon caractéristique de l'invention le processus opère une analyse de l'histogramme des valeurs ΔA (bloc 120), notamment pour discriminer entre un véritable bruit externe et une possible rupture de sonde. Si une rupture de sonde est avérée, alors une alerte est produite (bloc 122) et toute thérapie est désactivée (bloc 124), par sécurité.

Les Figures 6a et 6b montrent deux exemples d'histogrammes obtenus dans le cas où le dispositif a suspecte la présence d'un bruit (bloc 108) sur la base des seules analyses des paramètres ΔV et ΔA.

Cette situation peut recouvrir en fait deux cas différents, illustrés respectivement Figures 6a et 6b.

Dans le cas de la Figure 6a, il y a réellement présence d'un bruit externe de faible amplitude et de haute fréquence, ce qui se traduit sur l'histogramme par la présence de deux groupes clairement distincts (classes 1 à 3 et classe 10) sépares par un intervalle I supérieur à un seuil donné, par exemple deux groupes séparés de plus de I = 10 mV entre eux. Dans cet exemple, chaque classe correspond à un intervalle ΔA d'environ 1,8 mV et l'on observe sur l'histogramme deux groupes clairement distincts, l'un situé autour des intervalles 0-5 mV (classes 1 à 3, correspondant aux cycles bruités consécutifs) et l'autre autour de 18 mV (classe 10, correspondent au passage d'un cycle cardiaque normal à un bruit externe de faible amplitude et de haute fréquence et *vice versa).*

Dans le cas de la Figure 6b, la distribution des différentes classes est beaucoup plus aléatoire, et l'on note la présence de classes non vides dans une région supérieure B correspondent à une variabilité cycle-à-cycle supérieure à un seuil V1, par exemple V1 = 20 mV.

Dans cet exemple, chaque classe correspond à un intervalle ΔA de 2,5 mV. La présence de battements consécutifs présentant des différences d'amplitudes ΔA très élevées (classes 9 et 10 non vides, pour des valeurs supérieures à 20 mV) révèle une anomalie grave, résultant très probablement d'une rupture de sonde.

Les Figures 7a et 7b illustrent deux exemples de distribution des classes de l'histogramme, analysées au bloc 120, dans le cas où le dispositif n'a pas détecté de bruit et où l'on est en présence d'une tachyarythmie persistante avérée au bloc 114.

De façon caractéristique de l'invention, mais de manière subsidiaire, il est possible de qualifier la tachyarythmie, monomorphe ou polymorphe, par analyse de l'histogramme des valeurs ΔA.

Dans l'exemple de la Figure 7a, chaque classe correspond à un intervalle AA de 0,275 mV. Les classes non vides (classes 1 à 8 et 10) sont toutes situées dans une région C correspondant à une valeur inférieure à un seuil V2 donné, par exemple V2 = 3 mV, et on peut conclure à une tachyarythmie de caractère monomorphe.

Dans l'exemple de la Figure 7b, chaque classe correspond à un intervalle ΔA de 0,825 mV. Les classes non vides sont toutes réparties sur une étendue D allant jusqu'à un seuil maximal V3 (avec V2 < V3 < V1), par exemple V3 = 9 mV, et on peut conclure à une tachyarythmie de caractère polymorphe.

La Figure 8 illustre un exemple d'organigramme d'analyse de la répartition des différentes valeurs ΔA dans les classes de l'histogramme, qui n'est mise en œuvre que si Ton est toujours en épisode de tachyarythmie (bloc 200, correspondent au test 102 de la Figure 5).

On détecte la présence éventuelle d'un bruit de la manière expliquée plus haut (bloc 202, correspondent aux blocs 106 et 110 de la Figure 5).

En cas de suspicion de bruit, on recherche si l'histogramme comporte deux groupes distincts séparés d'un intervalle I supérieur à une valeur prédéterminée, par exemple séparés de plus de 10 mV avec des classes vides (bloc 204). Dans l'affirmative, on est en présence d'un bruit stable, de faible amplitude (bloc 206).

Pour mettre en évidence ces groupes, l'algorithme analyse le nombre de valeurs dans chaque classe. Si plusieurs classes consécutives, par exemple dix classes, sont vides et situées dans un domaine inférieur à une valeur maximale Max(ΔA), alors on peut confirmer la présence de groupes distincts dans l'histogramme, révélant un bruit stable de faible amplitude.

De plus, si la somme des Ni valeurs du premier groupe (celui des valeurs d'amplitude les plus faibles) est au moins x fois, par exemple x = 3 fois, supérieur à la somme des Ni valeurs du deuxième groupe (valeurs d'intervalle les plus élevées, qui correspondent au passage de la zone bruitée à un cycle cardiaque normal et *vice versa),* alors on peut conclure à un bruit stable de faible amplitude, et en plus de haute fréquence.

Si au bloc 204 l'analyse n'a pas permis d'observer deux groupes clairement distincts dans l'histogramme, alors on examine si la classe non vide la plus élevée Max(ΔA) est supérieure au seuil V1, par exemple V1 = 20 mV (bloc 208). Dans l'affirmative on est très vraisemblablement en présence d'un problème de sonde (bloc 210) ; dans le cas contraire, on ne confirme pas la rupture de sonde et on considère qu'il s'agit d'un bruit de nature indéterminée (bloc 212).

Si, à l'étape 202, on considère qu'il n'y a pas de bruit présent, alors l'algorithme recherche si la classe non vide la plus élevée Max(ΔA) est inférieure au seuil V2 (bloc 214).

Dans l'affirmative, on considère qu'il y a tachyarythmie monomorphe (bloc 216) et l'on marque l'épisode en conséquence.

Dans la négative, un autre test est effectué par rapport au seuil V3 (bloc 218): si Max(ΔA) dépasse ce seuil, on est en présence d'une tachycardie polymorphe (bloc 220) et on marque l'épisode en conséquence. Dans le cas contraire (bloc 202) il n'est pas possible de conclure et l'on ne marque pas l'épisode.

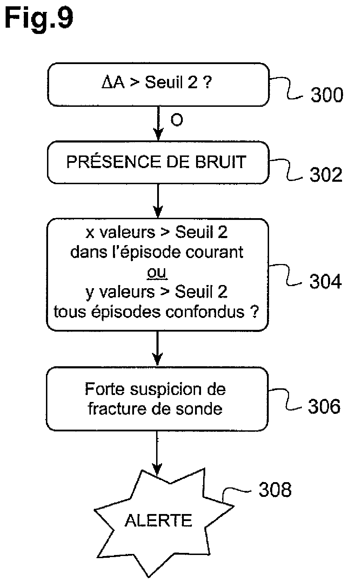

La Figure 9 illustre schématiquement les différentes étapes d'une analyse conduisant à conclure à une rupture de sonde, correspondent au bloc 122 de la Figure 5.

Outre la comparaison de la différence d'amplitude ΔA au seuil Seuil2, par exemple au seuil de 20 mV (bloc 300, correspondent eu bloc 110 de le Figure 5) si un bruit est suspecte (bloc 302, correspondent eu bloc 108 de la Figure 5), alors avant de déclencher l'alerte on applique des critères supplémentaires. Ainsi, ce n'est que si l'on trouve x valeurs de ΔA supérieures à Seuil2 dans l'épisode courant, ou y valeurs de ΔA supérieures à Seuil2 tous épisodes confondus, que l'on considère qu'il y a vraiment suspicion de rupture de sonde (bloc 306) et que l'on déclenche une alerte (bloc 308).

## Revendications

1. Un dispositif médical implantable actif (10), comportant :
- des moyens de recueil de signaux de dépolarisation d'origine ventriculaire, comprenant :
· des moyens aptes à recueillir au cours d'un même cycle cardiaque, concurremment sur des voies respectives distinctes, au moins deux signaux différents d'électrogramme endocavitaire, EGM, et en dériver au moins deux composantes temporelles (Vbip, Vuni) distinctes respectives ;
· des moyens aptes à combiner les au moins deux composantes temporelles (Vbip, Vuni) en au moins une caractéristique 2D paramétrique (VGM) représentative dudit cycle cardiaque, à partir des variations de l'une des composantes temporelles en fonction de l'autre ;
· des moyens aptes à dériver de la caractéristique 2D un descripteur géométrique représentatif de la caractéristique 2D , dans lequel le descripteur géométrique est l'angle moyen du vecteur vitesse (Vi) tangent à la caractéristique 2D, considéré en une pluralité de points respectifs (Pi) de cette caractéristique ; et
· des moyens aptes à déterminer la variation (ΔV) dudit descripteur géométrique au cours dudit cycle cardiaque ;
- des moyens d'application d'un choc de défibrillation ou d'impulsions de stimulation antitachycardique ;
- des moyens de détection d'épisodes de tachyarythmie ventriculaire ;
- des moyens de détection d'artefacts d'origine extracardiaque, aptes à comparer ladite variation du descripteur géométrique à un seuil prédé-terminé (Seuil1), et à inhiber les moyens d'application d'un choc de dé-fibrillation ou d'impulsions de stimulation antitachycardique lorsque cette variation du descripteur géométrique est supérieure audit seuil prédéterminé ;
**caractérisé en ce que** les moyens de détection d'artefacts d'origine extracardiaque comprennent en outre des moyens aptes, à chaque cycle d'un épisode de tachyarythmie détecté et lorsque la variation du descripteur géométrique n'est pas supérieure au seuil prédéterminé, à :
- mesurer et mémoriser les valeurs successives de la variation cycle-à-cycle (ΔA) de l'amplitude de l'un au moins desdits signaux EGM ;
- distribuer en une pluralité de classes lesdites valeurs de variation d'amplitude mémorisées, chaque classe correspondant à un intervalle d'amplitude ; et
- opérer une analyse statistique des effectifs de chaque classe de manière à délivrer sélectivement, en fonction d'au moins un critère prédéterminé appliqué à la distribution des variations d'amplitude dans les différentes classes, un indicateur de suspicion d'artefact d'origine extracardiaque et, en l'absence de détection d'artefact par les moyens de détection d'artefacts d'origine extracardiaque, un indicateur de type de tachyarythmie.

2. Le dispositif de la revendication 1, dans lequel les classes correspondent à des intervalles d'amplitude contigus et égaux.

3. Le dispositif de la revendication 1, dans lequel :
- ledit critère prédéterminé est la présence de deux classes non vides séparées par un intervalle prédéterminé (I) de classes vides consécutives, et
- l'indicateur est un indicateur de suspicion d'artefact par bruit d'origine extraventriculaire.

4. Le dispositif de la revendication 3, dans lequel intervalle prédéterminé (I) est un intervalle correspondant à un écart entre classes non vides d'au moins 10 mV.

5. Le dispositif de la revendication 1, dans lequel :
- ledit critère prédéterminé est la présence d'au moins une classe non vide correspondant à un intervalle d'amplitude supérieur à une première valeur limite (V1) donnée, et
- l'indicateur est un indicateur de suspicion d'artefact par rupture de sonde.

6. Le dispositif de la revendication 5, dans lequel ladite première valeur limite (V1) est d'au moins 20 mV.

7. Le dispositif de la revendication 5, dans lequel :
- ledit critère prédéterminé est le fait que, en l'absence de détection d'artefact par les moyens de détection d'artefacts d'origine extracardiaque, toutes les classes non vides sont des classes correspondant à des intervalles d'amplitude inférieure à une deuxième valeur limite (V2) don-née, inférieure à ladite première valeur limite (V1), et
- l'indicateur est un indicateur de tachyarythmie monomorphe.

8. Le dispositif de la revendication 7, dans lequel ladite deuxième valeur limite (V2) est d'au moins 2 mV.

9. Le dispositif de la revendication 7, dans lequel :
- ledit critère prédéterminé est le fait que, en l'absence de détection d'artefact par les moyens de détection d'artefacts d'origine extracardiaque, toutes les classes non vides sont réparties sur une étendue (D) allant jusqu'à une troisième valeur limite (V3) donnée, supérieure à ladite deuxième valeur limite (V2) et inférieure à ladite première valeur limite (V1), et
- l'indicateur est un indicateur de tachyarythmie polymorphe.

10. Le dispositif de la revendication 9, dans lequel ladite troisième valeur limite (V3) est d'au moins 5 mV.

## Patentansprüche

1. Aktive implantierbare medizinische Vorrichtung (10), umfassend :
- Mittel zum Sammeln von Depolarisationssignalen ventrikulären Ursprungs, umfassend :
- Mittel, die geeignet sind, im Verlauf ein und desselben Herzzyklus gleichzeitig auf jeweils unterschiedlichen Kanälen mindestens zwei unterschiedliche Signale des endokavitären Elektrogramms, EGM, zu sammeln und daraus mindestens zwei jeweils unterschiedliche zeitliche Komponenten (Vbip, Vuni) abzuleiten;
- Mittel, die geeignet sind, die mindestens zwei zeitlichen Komponenten (Vbip, Vuni) zu mindestens einem parametrischen 2D-Merkmal (VGM) zu kombinieren, das für den Herzzyklus repräsentativ ist, ausgehend von den Variationen einer der zeitlichen Komponenten in Abhängigkeit von der anderen ;
- Mittel, die geeignet sind, aus dem Merkmal 2D einen geometrischen Deskriptor abzuleiten, der für das Merkmal 2D repräsentativ ist, wobei der geometrische Deskriptor der mittlere Winkel des Geschwindigkeitsvektors (Vi) ist, der das Merkmal 2D tangiert, betrachtet an einer Vielzahl von jeweiligen Punkten (Pi) dieses Merkmals; und
- Mittel, die geeignet sind, die Veränderung (ΔV) des geometrischen Deskriptors im Verlauf des Herzzyklus zu bestimmen ;
- Mittel zum Anlegen eines Defibrillationsschocks oder von antitachykarden Stimulationsimpulsen ;
- Mittel zur Erkennung von Episoden ventrikulärer Tachyarrhythmie ;
- Mittel zur Erfassung von Artefakten extrakardialen Ursprungs, die geeignet sind, die Variation des geometrischen Deskriptors mit einem vorbestimmten Schwellenwert (Grenzwert H) zu vergleichen und die Mittel zur Anwendung eines Defibrillationsschocks oder von antitachykarden Stimulationsimpulsen zu hemmen, wenn diese Variation des geometrischen Deskriptors über dem vorbestimmten Schwellenwert liegt;
**dadurch gekennzeichnet, dass** die Mittel zur Erfassung von Artefakten extrakardialen Ursprungs außerdem Mittel umfassen, die geeignet sind, bei jedem Zyklus einer erfassten Tachyarrhythmie-Episode und wenn die Variation des geometrischen Deskriptors nicht größer als der vorbestimmte Schwellenwert ist, um :
- Messen und Speichern aufeinanderfolgender Werte der Zyklus-zu-Zyklus-(ΔA)-Änderung der Amplitude mindestens eines der EGM-Signale ;
- die gespeicherten Amplitudenänderungswerte in eine Vielzahl von Klassen zu verteilen, wobei jede Klasse einem Amplitudenintervall entspricht; und
- eine statistische Analyse der Anzahlen jeder Klasse durchzuführen, um in Abhängigkeit von mindestens einem vorbestimmten Kriterium, das auf die Verteilung der Amplitudenänderungen in den verschiedenen Klassen angewendet wird, selektiv einen Indikator für den Verdacht auf ein Artefakt extrakardialen Ursprungs und, wenn kein Artefakt durch die Mittel zur Erfassung von Artefakten extrakardialen Ursprungs erfasst wird, einen Indikator für den Typ der Tachyarrhythmie zu liefern.

2. Vorrichtung nach Anspruch 1, bei der die Klassen zusammenhängenden und gleichen Amplitudenintervallen entsprechen.

3. Vorrichtung nach Anspruch 1, wobei:
- das vorbestimmte Kriterium das Vorhandensein von zwei nicht leeren Klassen ist, die durch ein vorbestimmtes Intervall (I) von aufeinanderfolgenden leeren Klassen getrennt sind, und
- der Indikator ist ein Indikator für den Verdacht auf ein Artefakt durch Rauschen extraventrikulären Ursprungs.

4. Vorrichtung nach Anspruch 3, wobei das vorbestimmte Intervall (I) ein Intervall ist, das einem Abstand zwischen nicht leeren Klassen von mindestens 10 mV entspricht.

5. Vorrichtung nach Anspruch 1, wobei:
- das vorbestimmte Kriterium das Vorhandensein von mindestens einer nicht leeren Klasse ist, die einem Amplitudenintervall entspricht, das größer als ein gegebener erster Grenzwert (V1) ist, und
- der Indikator ist ein Indikator für den Verdacht auf ein Artefakt durch Sondenbruch.

6. Vorrichtung nach Anspruch 5, wobei der erste Grenzwert (V1) mindestens 20 mV beträgt.

7. Vorrichtung nach Anspruch 5, wobei:
- das vorbestimmte Kriterium die Tatsache ist, dass bei fehlender Artefakterkennung durch die Mittel zur Erkennung von Artefakten extrakardialen Ursprungs alle nicht leeren Klassen Klassen sind, die Intervallen mit einer Amplitude entsprechen, die kleiner ist als ein gegebener zweiter Grenzwert (V2), der kleiner ist als der erste Grenzwert (V1), und
- der Indikator ist ein monomorpher Tachyarrhythmieindikator.

8. Vorrichtung nach Anspruch 7, wobei der zweite Grenzwert (V2) mindestens 2mV beträgt.

9. Vorrichtung nach Anspruch 7, wobei:
- das vorbestimmte Kriterium die Tatsache ist, dass bei fehlender Artefakterkennung durch die Mittel zur Erkennung von Artefakten extrakardialen Ursprungs alle nicht leeren Klassen über einen Bereich (D) verteilt sind, der bis zu einem gegebenen dritten Grenzwert (V3) reicht, der größer ist als der zweite Grenzwert (V2) und kleiner als der erste Grenzwert (V1), und
- der Indikator ist ein Indikator für polymorphe Tachyarrhythmie.

10. Vorrichtung nach Anspruch 9, wobei der dritte Grenzwert (V3) mindestens 5 mV beträgt.

## Claims

1. An active implantable medical device (10) comprising:
- means for collecting depolarization signals of ventricular origin, comprising:
- means capable of simultaneously collecting, in the course of one and the same cardiac cycle, on respectively different channels, at least two different signals of the endocavitary electrogram, EGM, and of deriving therefrom at least two respectively different temporal components (Vbip, Vuni);
- means capable of combining said at least two temporal components (Vbip, Vuni) into at least one 2D parametric characteristic (VGM) representative of the cardiac cycle, starting from the variations of one of the temporal components as a function of the other;
- means adapted to derive from the feature 2D a geometric descriptor representative of the feature 2D, the geometric descriptor being the mean angle of the velocity vector (Vi) tangent to the feature 2D viewed at a plurality of respective points (Pi) of that feature; and
- means for determining the change (ΔV) of the geometric descriptor during the cardiac cycle ;
- means for applying a defibrillation shock or antitachycardia pacing pulses ;
- means for detecting episodes of ventricular tachyarrhythmia ;
- means for detecting artefacts of extracardiac origin, capable of comparing the variation of the geometric descriptor with a predetermined threshold (Threshold H) and of inhibiting the means for applying a defibrillation shock or antitachycardia pacing pulses when this variation of the geometric descriptor is above the predetermined threshold ; **characterized in that** said means for detecting artifacts of extracardiac origin further comprise means adapted, at each cycle of a detected tachyarrhythmia episode and when said variation of the geometric descriptor is not greater than said predetermined threshold value, for:
- measuring and storing successive values of the cycle-to-cycle (ΔA) change in amplitude of at least one of the EGM signals ;
- distributing the stored amplitude change values into a plurality of classes, each class corresponding to an amplitude interval; and
- performing a statistical analysis of the counts of each class to selectively provide, as a function of at least one predetermined criterion applied to the distribution of amplitude changes in the various classes, an indicator of suspicion of an artifact of extracardiac origin and, if no artifact is detected by the means for detecting artifacts of extracardiac origin, an indicator of the type of tachyarrhythmia.

2. The apparatus of claim 1, wherein the classes correspond to contiguous and equal amplitude intervals.

3. The apparatus of claim 1, wherein:
- the predetermined criterion is the presence of two non-empty classes separated by a predetermined interval (I) of successive empty classes, and
- the indicator is an indicator of suspected artifact due to noise of extraventricular origin.

4. The device of claim 3, wherein the predetermined interval (I) is an interval corresponding to a distance between non-empty classes of at least 10 mV.

5. The apparatus of claim 1, wherein:
- the predetermined criterion is the presence of at least one non-empty class corresponding to an amplitude interval greater than a given first threshold (V1), and
- the indicator is an indicator of suspected artifact due to probe breakage.

6. The device of claim 5, wherein the first threshold value (V1) is at least 20 mV.

7. The apparatus of claim 5, wherein:
- the predetermined criterion is the fact that, in the absence of artefact detection by the means for detecting artefacts of extracardiac origin, all the non-empty classes are classes corresponding to intervals having an amplitude smaller than a given second threshold (V2) which is smaller than the first threshold (V1), and
- the indicator is a monomorphic tachyarrhythmia indicator.

8. The device of claim 7, wherein the second threshold (V2) is at least 2mV.

9. The device of claim 7, wherein:
- the predetermined criterion is the fact that, in the absence of artefact detection by the means for detecting artefacts of extracardiac origin, all the non-empty classes are distributed over a range (D) extending up to a given third limit (V3) greater than the second limit (V2) and less than the first limit (V1), and
- the indicator is for polymorphic tachyarrhythmia.

10. The device according to claim 9, wherein the third limit value (V3) is at least 5 mV.
